# EUROPEAN PATENT APPLICATION

(11) **EP 1 625 869 A1**
(43) Date of publication of application: **15.02.2006**
(21) Application number: 04732819.0
(22) Date of filing: 13.05.2004
(51) Int. Cl.: A61M 25/10

(54) **BALLOON CATHETER AND METHOD OF MANUFACTURING THE SAME**

(30) Priority: 19.05.2003 JP 2003141213
(71) Applicant: KANEKA CORPORATION, Osaka-shi, Osaka 530-8288 (JP)
(72) Inventor: YAMAGUCHI, Youichi, Settsu-shi Osaka 566-0072 (JP); KOROGI, Mitsuharu, Settsu-shi, Osaka 566-0072 (JP)
(74) Representative: Gille Hrabal Struck Neidlein Prop Roos
(86) International application number: PCT/JP2004/006817
(87) International publication number: WO 2004/101057

(57) **Abstract**

A medical balloon catheter according to the present invention includes a balloon having a groove and/or a projection helicallyprovided relative to the longitudinal axis of the balloon. By such a structure, even after inflating the balloon once, for example, when the balloon is pushed into or removed through a severely stenosed lesion having a diameter smaller than that of the deflated balloon, the balloon can be easily wound more tightly. Therefore, pushing resistance can be reduced.

## Description

### Technical Field

The present invention relates to a balloon catheter used in percutaneous angioplasty (e.g., percutaneous transluminal angioplasty (PTA) or percutaneous transluminal coronary angioplasty (PTCA)) for dilating and treating stenosed or occluded coronary arteries, arteries of extremities, renal arteries, peripheral blood vessels, or the like. The present invention also relates to a method for producing a balloon and the balloon catheter.

### Background Art

Balloon catheters used in PTA or PTCA treatment each have a balloon at the distal end of a shaft and each are mostly composed of a flexible resin.

To perform PTCA treatment, a guiding catheter is inserted from a femoral artery through an aorta, and then the tip of the guiding catheter is positioned at the entrance of a coronary artery. Next, a guidewire is allowed to pass through a stenosed or occluded lesion in the coronary artery or the like. A balloon catheter is inserted along the guidewire. The balloon is placed at the lesion. A contrast medium or the like is introduced into the balloon to inflate the balloon. After dilation treatment of the lesion, the balloon is deflated by decompression, and the dilation catheter is removed from the body.

In recent years, balloon catheters have been required to be applicable to the highly stenosed, bent, and difficult lesions of blood vessels; and be capable of smoothly transferring balloons to lesions. Thus, the balloons and regions near the balloons have been softened. Furthermore, to reduce diameters, the shapes of balloons have been imparted by folding or the like. In addition, when a balloon that has been inflated once is transferred to another lesion or removed from the body, it is preferred that the balloon be deflated and automatically folded around the catheter shaft to reduce its size. For the purpose of this, various methods for imparting a shape of a balloon have been proposed.

For example, Japanese Unexamined Patent Application Publication No. 62-114565 discloses a method of folding a balloon along a single folding line in the longitudinal axis and then winding the folded balloon in four layers around a catheter shaft. Japanese Unexamined Patent Application Publication No. 3-92173 discloses a method of controlling folding by the difference of rigidity due to wall thickenss distribution on a balloon. PCT Japanese Translation Patent Publication No. 9-512190 discloses a method for imparting a shape of a balloon by disposing a cylindrical balloon in a moldbeing in the form of a regular tetragon in cross section and then heating the balloon while stretching. Japanese Patent No. 2671961 discloses a method for imparting a shape defined by at least three grooves extending along the longitudinal axis and wings to a balloon, the grooves and wings being alternately provided. Japanese Unexamined Patent Application Publication No. 2003-62080 discloses a method for imparting a shape of a balloon by forming a plurality of continuous grooves at least in the direction of the longitudinal axis and wings corresponding to the grooves using a die in advance, the grooves and wings corresponding to a scroll cross-section having grooves and projections, the number of wings being the same as that of the grooves. Japanese Unexamined Patent Application Publication No. 2002-263193 discloses a balloon having at least one flat face at a balloon taper or a projection and/or a groove at a balloon taper.

Various balloon structures are shown in the above-described Patent Documents. However, in any structure, when a balloon is inflated once, the balloon undergoes plastic deformation, thus losing a shape-memory effect. In deflation, a catheter is in the form of a plate including the longitudinal axis of the catheter (winging state. In this plate state, the length of a wing in the directionperpendicular to the longitudinal axis is larger than the diameter of a balloon being inflated, thus increasing resistance in removing the balloon from the body. Furthermore, a normal blood vessel or the like may be damaged) or a plurality of wings are projected. Thus, it is disadvantageously difficult to reduce the diameter. Therefore, in a known balloon catheter, it is difficult to provide a balloon catheter having desired recrossability.

### Disclosure of Invention

In view of the above-described problems to be solved by the present invention, the present invention provides a balloon catheter having satisfactory recrossability.

A medical balloon catheter according to the present invention includes a balloon having a groove and/or a projection helically provided on the balloon relative to the longitudinal axis of the balloon. By such a structure, even after inflating the balloon once, for example, when the balloon is pushed into or removed through a severely stenosed lesion having a diameter smaller than that of the deflated balloon, the balloon can be easily wound more tightly. Therefore, pushing resistance can be reduced.

In this case, preferably, the groove and/or the projection are helically provided on at least one balloon taper relative to the longitudinal axis of the balloon. According to this structure, for example, when the balloon is pushed into or removed through a difficult-to-pass area, such as a stenosed area, it is possible to more effectively reduce resistance.

A mold is preferably used in producing the balloon of the balloon catheter. Thereby, a balloon catheter can be easily produced in high yield and with stable quality.

By producing a balloon with laser irradiation, it is not necessary to produce a mold having a complex shape. Furthermore, the degree of freedom of the choice of the shape of the helical groove and/or projection is high.

A medical balloon catheter according to the present invention includes a balloon having a groove and/or a projection helicallyprovided relative to the longitudinal axis of the balloon. By such a structure, even after inflating the balloon once, for example, when the balloon is pushed into or removed from a severely stenosed lesion having a diameter smaller than that of the deflated balloon, the balloon canbe easilywoundmore tightly. Therefore, pushing or removing resistance can be reduced (excellent recrossability). In a known balloon catheter, various shapes of balloons have been used for improving recrossability. However, when a balloon is inflated once, the balloon undergoes plastic deformation, thus losing a shape-memory effect. As a result, it is difficult to achieve high recrossability. In an inventive balloon catheter having a groove and/or a projection helically provided on a balloon relative to the longitudinal axis, even if the balloon undergoes plastic deformation during inflation, it is assumed that since the balloon catheter can generate winding force that allows the balloon to be wound around the axis, high recrossability can be achieved. Furthermore, in a balloon catheter according to the present invention, a groove and/or a projection are helically provided relative to the longitudinal axis. Thus, for example, when a balloon is pushed toward the distal side in the axial direction by pushing force, the pushing force is dissipated and converted into winding force that allows the balloon to be wound around the axis. Therefore, it is conceivable that the balloon could be wound more tightly to improve recrossability.

In this case, preferably, the groove and/or the projection are helically provided on at least one balloon taper relative to the longitudinal axis of the balloon. According to this structure, for example, when the balloon is pushed into or removed through a difficult-to-pass area, such as a stenosed area, it is possible to more effectively reduce resistance. In view that the balloon can be pushed into a severely stenosed lesion, preferably, the groove and/or the projection are helically provided on a distal balloon taper, having a great effect in pushing the balloon, relative to the longitudinal axis of the balloon.

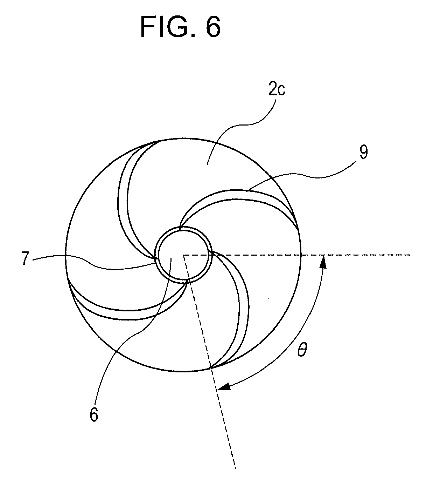

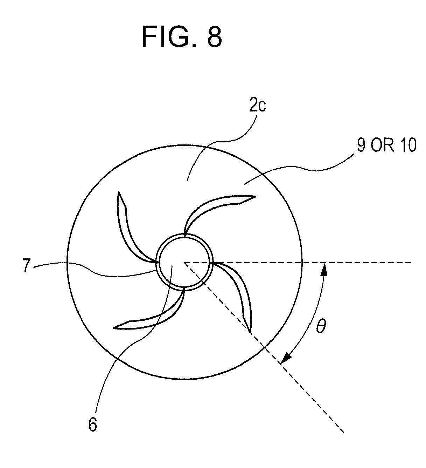

Preferably, the groove and/or the projection helically provided on a balloon taper relative to the longitudinal axis of the balloon are continuously provided at an angle ranging from 15° to 180° when viewed from the distal end, the angle being defined by a starting point and an ending point relative to the central axis (see Figs. 6 and 8). In this case, even after inflating the balloon once, the balloon is easily folded. Furthermore, for example, when the balloon is pushed into or removed through a severely stenosed lesion having a diameter smaller than that of the deflated balloon, the balloon can be easily wound more tightly. Therefore, pushing or removing resistance can be further reduced.

Preferably, the groove and/or the projection are provided on the distal balloon taper, the groove and/or the projection extending from the distal end to the proximal side. In this case, when the balloon is pushed into a severely stenosed lesion having a diameter smaller than that of the deflated balloon, the balloon can be easily wound more tightly. Therefore, pushing resistance can be further reduced (crossability at a severely stenosed lesion can be maximized).

With respect to the number of grooves and/or projections, a plurality of grooves and/or projections are preferably provided so that the diameter can be reduced when the balloon is deflated.

To be more specific, the number of grooves and/or projections is preferably 2 to 5 from the standpoint of the degree of efficiency and ease of production (when the number of grooves and/or projections is increased, it becomes difficult to produce the balloon).

To stably reproduce the shape of the balloon when the balloon is deflated (balloon can be stably wound), the width of the groove and/or projection is preferably 1 µm or more, more preferably 10 to 1,000 µm, and most preferably 10 to 250 µm. At a width of 1,000 µm or more, for example, when the balloon is formed by blow forming using a mold, the balloon bursts because of stress concentration. In addition, even when the balloon is formed by dipping, the resulting balloon has a nonuniform wall thickenss. In this way, the production of the balloon is adversely affected. At a width of 10 µm or less, the effect of the present invention on recrossability at a stenosed lesion after the balloon is inflated once is reduced. At a width exceeding 250 µm, the burst pressure of the balloon may be affected.

To stably reproduce the shape of the balloon when the balloon is deflated (balloon can be stably wound), the depth of the groove and/or the height of the projection is preferably 0.01 mm or more and more preferably 0.1 mm to 3.0 mm. At a depth and/or height exceeding 3.0 mm, the diameter may be increased when the balloon is folded.

To easily reproduce the winding of the balloon when the balloon is pushed into a stenosed lesion having a diameter smaller than that of the deflated balloon, the length of the groove and/or projection is preferably 0.1 mm to 4 mm.

On the other hand, the balloon having a groove and/or a projection helically provided relative to the longitudinal axis of the balloon is preferablyproduced using a mold, by laser heating, or the like. According to these methods, time and cost can be saved. Furthermore, a balloon catheter having the following properties can be produced: for example, when the balloon catheter is pushed into or removed from a severely stenosed lesion having a diameter smaller than that of the deflated balloon, even after inflating the balloon once, the balloon can be easily wound more tightly, and pushing or removing resistance is reduced.

Balloon catheters according to various embodiments of the present invention will be described below with reference to the drawings. The drawings are used for describing the present invention in detail. It is to be understood that the present invention is not limited thereto. In the drawings, the same reference numeral represents the same portion or an equivalent portion. Redundant description is not repeated, in some cases.

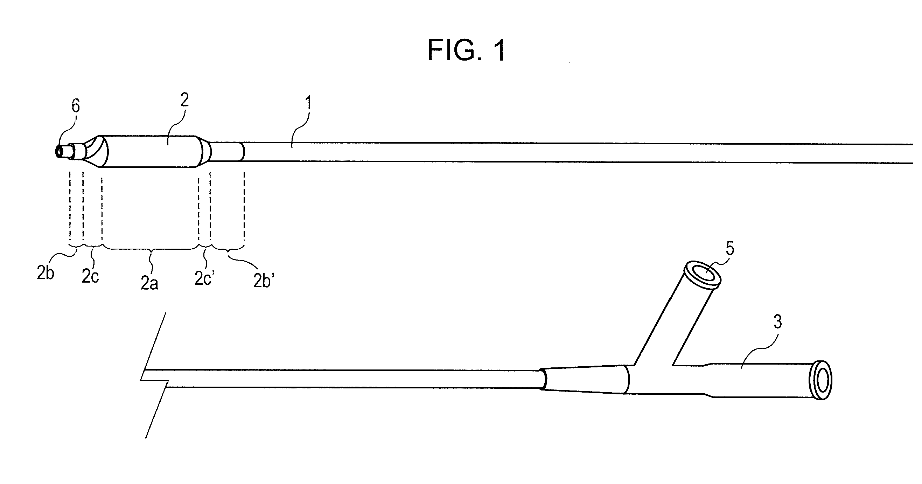

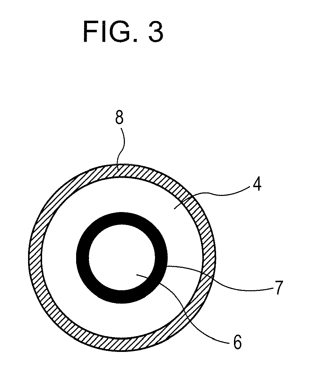

Figs. 1, 2, and 5 are each an appearance view showing a balloon catheter having a balloon according to an embodiment of the present invention. Fig. 3 is an enlarged view of the balloon catheter. The balloon 2 of the balloon catheter includes a distal sleeve 2b, a distal balloon taper 2c, a cylindrical midportion 2a, a proximal balloon taper 2c', and a proximal sleeve 2b'. Helical grooves 9 helically disposed relative to the longitudinal axis of the balloon are provided on the distal taper 2c.

With respect to a shaft structure, Fig. 1 shows an over-the-wire structure, and Fig. 2 shows a monorail structure. Each of these shafts usually includes an inflation lumen 4 and a guidewire lumen 6. For example, as shown in Fig. 3, a double-tube structure (coaxial type) in which a guidewire tube 7 including the guidewire lumen 6 is inserted and coaxially disposed in an inflation tube 8 including the inflation lumen 4 may be used. Alternatively, as a balloon catheter disclosed in Japanese Unexamined Patent Application Publication No. 7-178175, a structure (biaxial type) in which the inflation lumen 4 and the guidewire lumen 6 are not coaxially disposed may be used (Fig. 4). Various structures other than these may be used for the shaft of the balloon catheter according to the present invention within the scope of the gist of the present invention.

The base of the shaft may be composed of a relatively hard material at the proximal side. Examples of the material include metals such as Ni-Ti, stainless steel (SUS), brass, aluminum or an alloy thereof; and resins having relatively high rigidity, for example, polyimides, polycarbonates, polyamides, and poly(vinyl chlorides). Examples of the material used at the distal side include polystyrenes, polyolefins, polyesters, polyamides, polyurethanes, polypropylenes, and polyvinyl chlorides; elastomers of these polymers; and mixtures containing a plurality of these polymer. In addition, the shaft maybe formed of a laminated tube composed of these materials.

Fig. 6 is a schematic front view of the balloon catheter when viewed from the distal end. Each of the grooves 9 is helically, continuously provided relative to the longitudinal axis of the balloon from the distal end to the proximal side of the distal balloon taper 2c. By providing the grooves helically provided on the balloon relative to the longitudinal axis of the balloon, even after inflating the balloon once, for example, when the balloon is pushed into or removed from a severely stenosed lesion having a diameter smaller than that of the deflated balloon, the balloon can be easily wound more tightly. Therefore, pushing or removing resistance can be reduced.

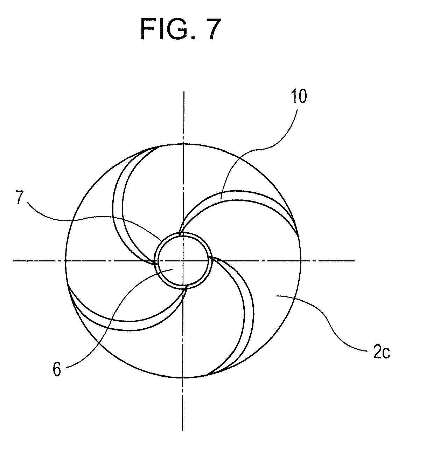

Fig. 7 is a schematic front view of a balloon catheter according to an embodiment of the present invention when viewed from the distal end. Each projection 10 is helically, continuously provided relative to the longitudinal axis of the balloon from the distal end to the proximal side of the distal balloon taper 2c. By providing the projections on the balloon, the projections being helically disposed relative to the longitudinal axis of the balloon, even after inflating the balloon once, for example, when the balloon is pushed into or removed from a severely stenosed lesion having a diameter smaller than that of the deflated balloon, the balloon can be easily wound more tightly. Therefore, pushing or removing resistance can be reduced.

Fig. 8 is a schematic front view of a balloon catheter according to an embodiment of the present invention when viewed from the distal end. As shown in Fig. 8, grooves and/orproj ections need not be provided on the entire distal balloon taper 2c in the direction of the longitudinal axis. If the grooves or projections provided on the distal balloon taper from the distal end to the proximal side interfere with the assembly of the catheter, the grooves or projections may be partially provided on the taper. However, to achieve high recrossability, the grooves or projections are preferably provided on the entire distal balloon taper in the direction of the longitudinal axis.

In addition to the description above, with respect to the length, width, and depth (height) of the groove and/or the projection, when a plurality of grooves and/or projections are provided on one balloon, the lengths, widths, and depths (heights) of the grooves and/or projections may be the same or different. In other words, the grooves or projections may have any shape. However, from the standpoint of the difficulty of fabrication and cost, the shapes are preferably the same and linear.

As a method for forming the grooves and/or projections helicallyprovided relative to the longitudinal axis of the balloon, a method in which the grooves and/or projections are formed simultaneously with the formation of the balloon may be employed. Alternatively, after a balloon having no groove and/or projection is formed, the groove and/or projection may be formed separately. To be specific, the following method for forming the groove and/or projection may be applied: a method in which a mold is used in balloon blowing, a dipping method, or a method in which physical energy such as a laser is used. Furthermore, any of various methods other than these methods may be employed for forming the groove and/or projection.

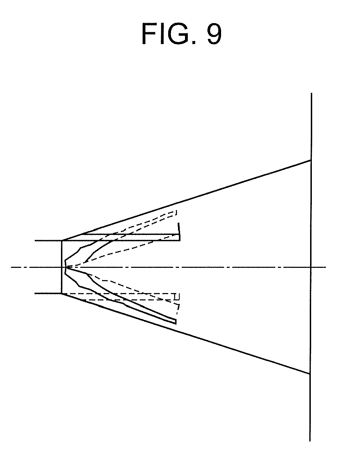

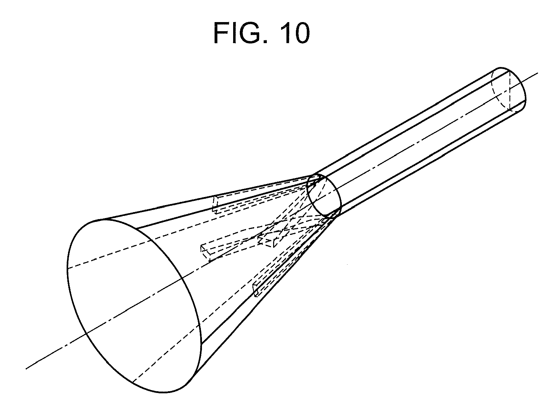

However, from the standpoint of the reproducibility of the balloon shape, folded form, and wrapping performance, time cost, and the like in mass production, a method of using a mold in balloon blowing is preferable. An example of a mold used for forming a balloon having a groove and/or a projection is shown in Fig. 9 (perspective side view of a taper) and Fig. 10 (perspective view of the taper and the sleeve). This mold is used for producing a balloon, as shown in Fig. 8, having the grooves 9 that are not entirely provided on the balloon taper 2c across the longitudinal direction.

On the other hand, the groove and/or the projection may be formed by applying thermal energy to a balloon that has already been formed or by irradiating the balloon with a laser. In this case, it is not necessary to produce a mold having a complex shape. Furthermore, the degree of freedom of the choice of the shape of the helical groove and/or projection is high.

With respect to the size of the balloon 2, the maximum outer diameter of the cylindrical midportion 2a in inflating the balloon is preferably about 1 mm to 20 mm and more preferably about 1 to 10 mm. The length of the cylindrical midportion of the balloon is preferably about 5.0 mm to 70 mm and more preferably about 10 mm to 50 mm. The total length of the balloon is preferably about 10 to 100 mm and more preferably about 15 mm to 70 mm. The wall thickenss of the balloon is preferably about 5 µm to 80 µm and more preferably about 10 µm to 50 µm. The wall thickenss may be substantially uniform or may be nonuniform.

The balloon is preferably composed of a material having a certain degree of plasticity so as to dilate a stenosed lesion. Examples of the material include polyolefins, polyolefin elastomers, polyesters, polyester elastomers, polyamides, polyamide elastomers, polyurethane, polyurethane elastomers, fluorocarbon resins, ionomers, and latex rubbers. Furthermore, a mixture or a laminated material of these may be used. It is understood that a material containing fillers such as metal particles or plastic fibers may be used.

### Brief Description of the Drawings

Fig. 1 is a schematic side view of an over-the-wire balloon catheter according to the present invention.

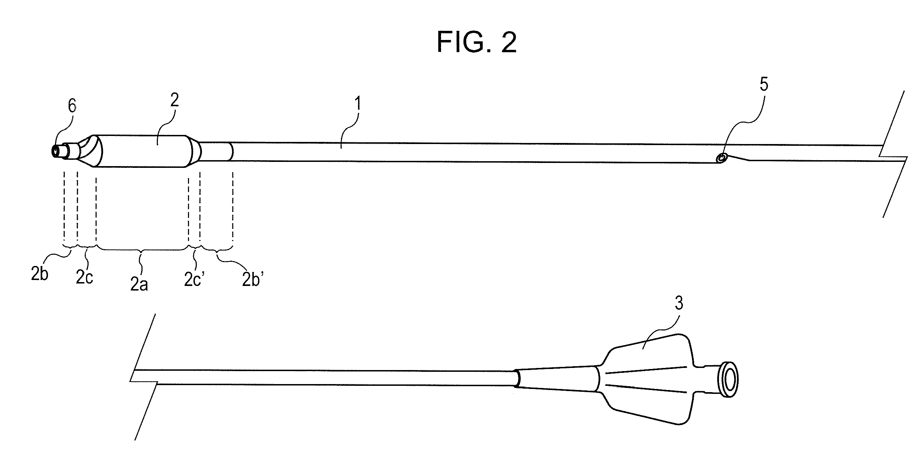

Fig. 2 is a schematic side view of a monorail balloon catheter according to the present invention.
Fig. 3 is a schematic cross-sectional viewof a coaxial-type shaft of a balloon catheter according to the present invention.

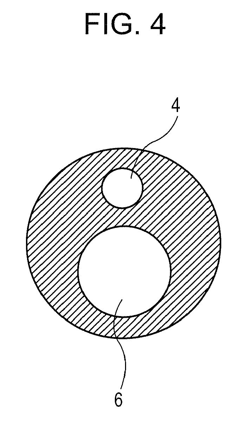

Fig. 4 is a schematic cross-sectional viewof abiaxial-type shaft of a balloon catheter according to the present invention.

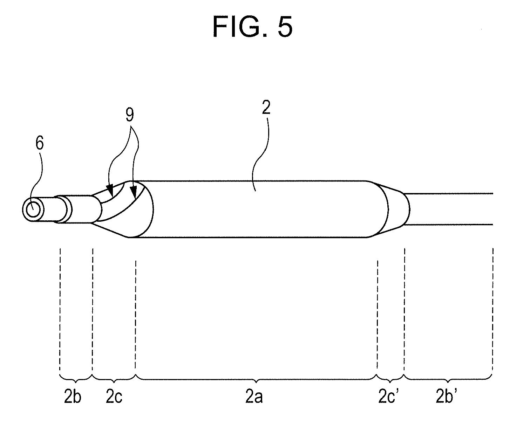

Fig. 5 is a schematic side view of the balloon of a balloon catheter according to the present invention, the balloon having grooves on the entire distal balloon taper, and the grooves being helically disposed relative to the longitudinal axis.
Fig. 6 is a schematic front view of the balloon, having grooves, of a balloon catheter according to the present invention, the grooves continuously provided from the end of the distal balloon taper toward the proximal side being tangential to the external wall of the distal balloon sleeve, and the grooves being helically provided relative to the longitudinal axis and continuously provided to the cylindrical midportion of balloon. θ is an angle defined by a starting point and an ending point of the groove and/or the projection provided on the balloon taper relative to the central axis.
Fig. 7 is a schematic front view of the balloon, having projections, of a balloon catheter according to the present invention, the projections continuously provided from the end of the distal balloon taper toward the proximal side being tangential to the external wall of the distal balloon sleeve, and the projections being helically provided relative to the longitudinal axis and continuously provided to the cylindrical midportion.
Fig. 8 is a schematic front view of the balloon, having grooves and/or projections, of a balloon catheter according to the present invention, the grooves and/or projections continuously provided from the end of the distal balloon taper toward the proximal side being tangential to the external wall of the distal balloon sleeve, and the grooves and/or projections being helically, continuously provided relative to the longitudinal axis and being provided to the intermediate position of the taper toward the cylindrical midportion. θ is an angle defined by a starting point and an ending point of the groove and/or the projection provided on the balloon taper relative to the central axis.
Fig. 9 is a schematic side perspective view of a mold used in forming the balloon, having grooves, of a balloon catheter according to the present invention, the grooves continuously provided from the end of the distal balloon taper to the proximal side being tangential to the external wall of the distal balloon sleeve, and the grooves being helically, continuously provided relative to the longitudinal axis and being partially provided on the taper toward the cylindrical midportion.
Fig. 10 is a schematic perspective view of a mold used in forming the balloon, having grooves, of a balloon catheter according to the present invention, the grooves continuously provided from the end of the distal balloon taper to the proximal side being tangential to the external wall of the distal balloon sleeve, and the grooves being helically, continuously provided relative to the longitudinal axis and being partially provided on the taper toward the cylindrical midportion.

### Reference Numerals

Reference numeral 1 represents a shaft, reference numeral 2 represents a balloon, reference numeral 2a represents the cylindrical midportion of the balloon, reference numeral 2b represents the distal sleeve of the balloon, reference numeral 2b' represents the proximal sleeve of the balloon, reference numeral 2c represents the distal taper of the balloon, reference numeral 2c' represents the proximal taper of the balloon, reference numeral 3 represents a manifold, reference numeral 4 represents an inflation lumen, reference numeral 5 represents an inflation port, reference numeral 6 represents a guidewire lumen, reference numeral 7 represents a guidewire tube, reference numeral 8 represents a inflation tube, reference numeral 9 represents a groove, reference numeral 10 represents a projection, and θ represents an angle defined by a starting point and an ending point of the groove and/or the projection provided on the balloon taper relative to the central axis (Figs. 6 and 8).

### Best Mode for Carrying Out the Invention

Specific examples and Comparative example according to the present invention will be described in detail below. However, it is to be understood that the present invention is not limited thereto.

### (Example 1)

A tubular parison (inner diameter: 0.43 mm, oute rdiameter: 0.89 mm) was produced by extrusion molding with a polyamide elastomer (trade name: PEBAX7233SA01, manufactured by Elf Atochem, Inc.). Next, a balloon having an external diameter of 3.0 mm at the cylindrical midportion was produced by biaxial stretching blow forming with the resulting parison using a balloonmold capable of forming grooves as shown in Fig. 6. The grooves were provided from the distal end of the distal balloon taper to the proximal side. The number of grooves was four. The grooves each had a width of 200 µm and a depth of 100 µm. θ was 60°.

A guidewire tube (inner diameter: 0.42mm, outer diameter: 0.56 mm) and an inflation tube (inner diameter: 0.71 mm, outer diameter: 0.88 mm), which were used as tubes at the distal side of the shaft base and were composed of a polyamide elastomer (trade name: PEBAX7233SA01, manufactured by Elf Atochem, Inc.), were produced by extrusion molding. These tubes and a tube (inner diameter: 0.50 mm, outer diameter: 0.66 mm), which was used as a tube at the proximal side of the shaft base, composed of SUS316 stainless steel were used to produce a coaxial-type monorail balloon catheter.

### (Example 2)

A balloon catheter was produced as in Example 1, except that a balloon mold capable of forming helical grooves from the distal end of the distal balloon taper to the intermediate portion of the distal balloon taper, as shown in Fig. 8, was used. In this Example, the number of grooves was four. The grooves each had a width of 200 µm and a depth of 100 µm or less. θ was 60°.

### (Comparative example)

A balloon catheter was produced using a balloon mold as in Examples 1 and 2, except that the helical grooves were not provided on the balloon.

The balloon catheters produced in Examples 1 and 2 and Comparative example were evaluated by the following method.

### (Evaluation)

A simulated aorta and a guiding catheter were set in a vessel filled with physiological saline at 37°C. The tip of the guiding catheter was connected to a simulated small-diameter tube 1.50 mm in inner diameter composed of polyethylene, the tube simulating the stenosed lesion of a coronary artery. A balloon catheter was inserted in the guiding catheter with a guidewire in advance. The guidewire was disposed so as to protrude 100 mm from the distal end of the balloon catheter. A mixture of a contrast medium and physiological saline was introduced into the balloon catheter to 14 atm with an indef lator, and the inflated balloon was maintained for 30 seconds. Then, the balloon was deflated rapidly. The balloon catheter was pushed into the simulated small-diameter tube at a rate of 10 mm/sec with a sliding table. The maximum load generated was measured at n = 5 in each Example with a digital force gauge. Table 1 shows the evaluation results.

**Table 1**

| | Comparative example | Example 1 | Example 2 |
|---|---|---|---|
| 1 | impassable | 30.8 | 33.3 |
| 2 | Impassable | 27.6 | 31.5 |
| 3 | 52.4 | 28.0 | 29.9 |
| 4 | impassable | 34.2 | 37.6 |
| 5 | 68.9 | 30.5 | 38.1 |

| | | | |
|---|---|---|---|
| *unit: gram-force (gf) | | | |

As shown in Table 1, three of the five balloon catheters produced in Comparative example were impassable, whereas all of balloon catheters produced in Examples were passable. Pushing loads generated when the balloon catheters produced in Examples were passed were clearly stably low compared with those in Comparative example. Furthermore, the method for producing the balloon was simple and the balloon was relatively easily formed in very high yield.

### Industrial Applicability

As has been described above, since a balloon catheter according to the present invention has grooves and/or projections helically provided relative to the longitudinal axis of the balloon, even after inflating the balloon once, for example, when the balloon is pushed into or removed from a severely stenosed lesion having a diameter smaller than that of the deflated balloon, the balloon can be easily wound more tightly. Therefore, pushing or removing resistance can be reduced.

Furthermore, with respect to a production process, a balloon can be easily produced using a mold in high yield and with stable quality. In addition, by irradiating a balloon with a laser, it is not necessary to produce a mold having a complex shape. Furthermore, the degree of freedom of the choice of the shape of the helical groove and/or projection is high.

## Claims

1. A medical balloon catheter comprising a balloon having a groove and/or a projection helically provided on the balloon relative to the longitudinal axis of the balloon.

2. The medical balloon catheter according to claim 1, wherein the groove and/or the projection are helically provided on at least one balloon taper relative to the longitudinal axis of the balloon.

3. The medical balloon catheter according to claim 2, wherein the groove and/or the projection helically provided on a balloon taper relative to the longitudinal axis of the balloon are continuously provided at an angle ranging from 15° to 180° when viewed from the distal end, the angle being defined by a center-line connecting the center and a starting point and another-line connecting the center and an ending point.

4. The medical balloon catheter according to claim 2 or 3, wherein the groove and/or the projection are helically provided on a distal balloon taper relative to the longitudinal axis of the balloon, said the groove and/or the projection being extending from the distal end to the proximal side.

5. The medical balloon catheter according to any one of claims 1 to 4, wherein a plurality of grooves and/or projections are helically provided on a balloon taper relative to the longitudinal axis of the balloon.

6. The medical balloon catheter according to any one of claims 1 to 5, wherein the number of the grooves and/or projections helically provided on a balloon taper relative to the longitudinal axis of the balloon is 2 to 5.

7. The medical balloon catheter according to any one of claims 1 to 6, wherein the width of the groove and/or the projection helically provided on a balloon taper relative to the longitudinal axis of the balloon varies in the direction of the longitudinal axis of the balloon.

8. The medical balloon catheter according to any one of claims 1 to 7, wherein the width of the groove and/or the projection helically provided on a balloon taper relative to the longitudinal axis of the balloon is 1 µm or more.

9. The medical balloon catheter according to any one of claims 1 to 8, wherein the width of the groove and/or the projection helically provided on a balloon taper relative to the longitudinal axis of the balloon is 10 µm to 1,000 µm.

10. The medical balloon catheter according to any one of claims 1 to 9, wherein the depth of the groove and/or the height of the projection helically provided on a balloon taper relative to the longitudinal axis of the balloon is 0.01 m or more.

11. The medical balloon catheter according to any one of claims 1 to 10, wherein the depth of the groove and/or the height of the projection helically provided on a balloon taper relative to the longitudinal axis of the balloon is 0.1 mm or more to 3.0 mm or less.

12. The medical balloon catheter according to any one of claims 1 to 11, wherein the length of the groove and/or the projection helically provided on a balloon taper relative to the longitudinal axis of the balloon is 0.1 mm or more to 4.0 mm or less.

13. A method for producing a balloon catheter including a balloon having a groove and/or a projection helically provided on a balloon taper relative to the longitudinal axis of the balloon, the method comprising forming the balloon with a mold.

14. A method for producing a balloon catheter including a balloon having a groove and/or a projection helically provided on a balloon taper relative to the longitudinal axis of the balloon, the method comprising applying thermal energy to a preliminary formed balloon to form a groove and/or a projection helically provided on the balloon relative to the longitudinal axis of the balloon.

15. A method for producing a balloon catheter including a balloon having a groove and/or a projection helically provided on a balloon taper relative to the longitudinal axis of the balloon, the method comprising irradiating a preliminally formed balloon with a laser to form a groove and/or a projection on a balloon taper helically provided relative to the longitudinal axis of the balloon.

16. The method for producing the balloon catheter according to any one of claims 13 to 15, wherein the groove and/or the projection to be helically provided on a balloon taper relative to the longitudinal axis of the balloon are formed on at least one balloon taper.

17. The method for producing the balloon catheter according to any one of claims 13 to 15, wherein the groove and/or the projection to be helically provided on a balloon taper relative to the longitudinal axis of the balloon are formed on a distal balloon taper, the groove and/or the projection extending from the distal end to the proximal side.
